# EUROPEAN PATENT APPLICATION

(11) **EP 2 607 342 A1**
(43) Date of publication of application: **26.06.2013**
(21) Application number: 11194988.9
(22) Date of filing: 21.12.2011
(51) Int. Cl.: C07C 41/22, C07C 67/31, C07C 43/12, C07C 69/708

(54) **Process for the manufacture of substituted malonic acid derivative compounds and of Sevoflurane**

(71) Applicant: SOLVAY SA, 1120 Bruxelles (BE)
(72) Inventor: Braun, Max, Josef, 30900 Wedemark (DE)
(74) Representative: Mross, Stefan P.M.

(57) **Abstract**

Process for the manufacture of a substituted malonic acid derivative of formula (I) or (II):

R₁OOC-CH(OCH₂F)-COOR₂ (I)

R₃HNOC-CH (OCH₂F)-CONHR₄ (II)

by reaction of a malonic acid derivative of formula (III) or (IV):

R₁OOC-CHOH-COOR₂ (III)

R₃HNOC-CHOH-CONHR₄ (IV)

with at least one CH₂-electrophile selected from the group consisting of (a) bis(fluoromethyl)ether and (b) formaldehyde activated with HF and at least one additional activator. The compounds of formula (I) and (II) can be fluorinated to produce Sevoflurane.

## Description

The present invention relates to a process for preparing Sevoflurane, CF₃-CH(OCH₂F)-CF₃. It also relates to a process for the synthesis of certain substituted malonic acid derivative compounds.

Sevoflurane is an important volatile anesthetic agent particularly suited for administration to patients during outpatient surgery. Sevoflurane is known to provide patients with a rapid rate of recovery from the anesthesia. An additional advantage of this anesthetic agent is that it can be used as an induction agent since it is not pungent and allows a rapid and smooth induction without breath holding or laryngospasm as may occur with other inhalation agents. A smooth uneventful induction is especially valuable for pediatric anesthesia where the use of intravenous induction agents can result in numerous problems and is often contraindicated.

Many of the commercial processes for the preparation of Sevoflurane make use of the chemical intermediate CF₃-CH(OH)-CF₃ (HFIP), as starting material. In most of these processes, unreacted HFIP may remain in the product mixture. It is known in the art that HFIP is difficult to remove from the crude Sevoflurane product. WO-A- 2010/125899 discloses the manufacture of Sevoflurane by reacting hexafluoroisopropanol with bis(fluoromethyl)ether in the presence of a strong acid.

This application now relates to an improvement of applicant's inventions disclosed in WO - A - 2011018466, the whole content of this application being incorporated herein by reference for all purposes.

The prior invention discloses a particular embodiment, wherein a substituted malonic acid derivative of formula (I) or (II):

R₁OOC-CH (OCH₂X)-COOR₂ (I)

R₃HNOC-CH (OCH₂X)-CONHR₄ (II)

is manufactured by the reaction of the hydroxyl malonic acid derivative of formula (VIII) or (IX): R₁OOC-CH(OH)-COOR₂ (VIII) or R₃HNOC-CH (OH)-CONHR₄ (IX) wherein R₁, R₂ R₃ and R₄ are as defined above, with a CH₂-electrophile in the presence of a leaving group transfer agent may be carried out additionally in the presence of a strong acid. The strong acid may be selected for example from the group consisting of fuming sulfuric acid, fuming nitric acid and hydrogen halides such as in particular hydrogen chloride, hydrogen fluoride.

In a first specific embodiment, the strong acid is a fuming sulfuric acid.

In a second specific embodiment, the strong acid is hydrogen chloride or hydrogen fluoride.

It has now been found a process for the manufacture of Sevoflurane, CF3-CH(OCH₂F)-CF₃ and synthesis intermediates thereof which allows for improved yield, high efficiency and lower manufacturing cost in particular compared to the known processes whereby HFIP is used as chemical intermediate.

The invention consequently relates to a process for the manufacture of a substituted malonic acid derivative of formula (I) or (II):

R₁OOC-CH(OCH₂F)-COOR₂ (I)

R₃HNOC-CH (OCH₂F)-CONHR₄ (II)

wherein
- R₁, R₂ equal to or different from each other, are independently selected from H, an alkyl group having from 1 to 10 carbon atoms which is optionally substituted by at least one halogen atom, an aralkyl group or an aryl group;
- R₃, R₄ equal to or different from each other, are independently selected from H, an alkyl group having from 1 to 10 carbon atoms which is optionally substituted by at least one halogen atom, an aralkyl group or an aryl group; by reaction of a malonic acid derivative of formula (III) or (IV):

   R₁OOC-CHOH-COOR₂ (III)

   R₃HNOC-CHOH-CONHR₄ (IV)

   wherein
- R₁, R₂ R₃ and R₄ are as defined above, with at least one CH₂-electrophile selected from the group consisting of (a) bis(fluoromethyl)ether (CH₂FOCH₂F) and (b) formaldehyde activated with HF and at least one additional activator.

Surprisingly, the process according to the invention makes possible an improved yield of malonic acid derivatives while having simultaneously advantages concerning the purification of Sevoflurane.

For the purpose of the present invention, the term "aryl group" refers to an aromatic ring group such as phenyl and napthyl, and phenyl and napthyl substituted by at least 1 halogen atom.

For the purpose of the present invention, the term "aralkyl group" refers to an aromatic ring group substituted with alkyl groups such as tolyl, biphenylyl, etc.

Preferably, in the process according to the invention, R₁, R₂, R₃ and R₄ equal to or different from each other, are often independently selected from H, a C1-C4 alkyl group, optionally substituted by at least 1 halogen atom, an aryl group, for example, phenyl. More preferably, R₁, R₂, R₃ and R₄ equal to or different from each other, are independently from each other H, a linear or branched C1-C4 alkyl group optionally substituted by at least 1 halogen atom, and particularly preferably R₁, R₂, R₃ and R₄ are independently from each other H, methyl, ethyl, n-propyl or isopropyl each optionally substituted by at least 1 halogen atom, very preferably H, a methyl or an ethyl group each optionally substituted by at least 1 halogen atom. Most preferably, R₁ and R₂ are ethyl.

In another aspect, R₁ and R₂ are H.

In general, the reaction is performed in the liquid phase.

In the process according to the invention, the pressure and the temperature are therefore usually selected as such that the reaction mixture remains in the liquid phase.

The process according to the present invention generally comprises carrying out the reaction at a temperature equal to or higher than 0°C, preferably equal to or higher than 40°, more preferably equal to or higher than 50°C. It is generally carried out at a temperature equal to or lower than 150°C, preferably at a temperature equal to or lower than 120°C, more preferably a temperature equal to or lower than 100°C. A temperature ranging from 50 to 70 °C is most preferred.

In the process according to the invention, the molar ratio between formaldehyde or bis(fluoromethyl)ether and malonic acid derivative of formula (III) or (IV) is generally from 1,0 to 5,0, preferably from 1,1 to 2,0.

In one aspect, the process according to the present invention comprises carrying out the reaction in the presence of a solvent, in particular a non-protic organic solvent. The solvent to be used may, for example, be a polar solvent such as an amide type solvent, in particular dimethyl formamide or dimethyl acetamide, anitrile, in particular acetonitrile, a sulphur containing solvent, in particular dimethylsulfoxide or sulfolane, a halogenated hydrocarbon such as methylene chloride, chloroform or ethylene dichloride ; or an ether such as diethyl ether, dibutyl ether, dioxane or tetrahydrofuran.

Fluorinated solvents, for example a fluorinated hydrocarbon such as in particular 1,1,1,3,3-pentafluorobutane, or a fluorinated ether such as Sevoflurane are preferred. Particularly preferred solvents are selected from dimethyl formamide, sulfolane, 1,1,1,3,3-pentafluorobutane or Sevoflurane. These solvents may be used alone or in combination as a mixture. If appropriate, the solvent is used usually in an amount of from 50 to 99 by weight, preferably from 60 to 99 % by weight, more preferably from 75 to 99% by weight of the solvent relative to the total weight of the reaction medium.

When the CH₂-electrophile is bis(fluoromethyl)ether, the reaction is generally carried out in the presence of at least one additional activator.

When an additional activator is used in the process according to the invention, it is generally a strong acid other than HF. The strong acid may be selected for example from the group consisting of sulfuric acid, in particular fuming sulfuric acid, nitric acid, in particular fuming nitric acid and hydrogen chloride. In another aspect, the strong acid is a perfluoroalkylsulphonic acid, such as in particular trifluoromethylsulphonic acid or p-trifluoromethylbenzenesulphonic acid. The additional activator is preferably sulphuric acid. In the process according to the invention, the molar ratio between additional activator and formaldehyde or bis(fluoromethyl)ether is generally from 0,001 To 0,5, preferably from 0,002 to 0,01.

When the CH₂-electrophile is formaldehyde activated with HF and at least one additional activator, the molar ratio HF/formaldehyde is generally from 1 to 10, preferably from 2 to 5.

The invention also concerns a process for the manufacture of Sevoflurane (CF₃-CH(OCH₂F)-CF₃) which comprises (a) the manufacture of a substituted malonic acid derivative of formula (I) or (II) according to the process according to the invention described herein before and (b) the fluorination of the malonic acid derivative of formula (I) or (II) obtained in step (a). In a particular embodiment, when R1 and R2 are alkyl, as described above, it is preferred to saponify the ester groups in compounds of formulae (I) to obtain a compound of formula (I) wherein R1 and R2 are H and to use the so obtained compound in step (b) of the manufacture of Sevoflurane. The fluorination can be suitably carried out according to the processes disclosed in W02011018466. The fluorination is preferably carried out using SF4 as fluorination reagent.

The processes according to the invention and the particular embodiments thereof can be performed in any suitable reactor, e.g. in an autoclave.

The processes according to the invention for the manufacture of the intermediate substituted malonic acid derivative and for the reaction of said intermediate to form Sevoflurane and the particular embodiments thereof can be carried out batchwise or continuously. In the case of a batchwise introduction of the relevant reactants, the molar ratios referred to herein concern the initial molar ratio. In the case of a continuous introduction of the relevant reactants, the molar ratios referred to herein concern the molar ratio between the respective continuous feeds.

Should the disclosure of any patents, patent applications, and publications which are incorporated herein by reference conflict with the description of the present application to the extent that it may render a term unclear, the present description shall take precedence.

The examples here after are intended to illustrate the invention without however limiting it.

### Examples

### Example 1 - Synthesis of EtOOC-CH(OCH₂F)-COOEt by reaction of EtOOC-CH(OH)-COOEt with bis(fluoromethyl ether)

A solution of 0,3 moles bis-fluoromethylether in 25 ml 1,1,1,3,3-pentafluorobutane is prepared and 0,1 mole EtOOC-CH(OH)-COOEt, dissolved in 25 ml 1,1,1,3,3-pentafluorobutane is added at 20°C. Stirring is continued for 1h. The product EtOOC-CH(OCH₂F)-COOEt is isolated by distillation.

### Example 2 - Synthesis of EtOOC-CH(OCH₂F)-COOEt by reaction with formaldehyde/HF/H2SO4

A solution of 0,3 moles bis-fluoromethylether in 25 ml methylene chloride is prepared by dissolving 0.3 moles paraformaldehyde in 25ml methylene chloride and addition of 1,5 moles anhydrous HF dissolved in 25 ml 1,1,1,3,3-pentafluorobutane under ice cooling. To this solution, 0,1 mole EtOOC-CH(OH)-COOEt, dissolved in 25 ml 1,1,1,3,3-pentafluorobutane is added at 20°C. Finally, 0,01 mole of H2SO4 diluted in 1 mL 1,1,1,3,3-pentafluorobutane is added dropwise. Stirring is continued for 1h. The product EtOOC-CH(OCH₂F)-COOEt is isolated by distillation.

### Example 3 - Synthesis of Sevoflurane from EtOOC-CH(OCH₂F)-COOEt

0,05 mole EtOOC-CH(OH)-COOEt obtained from example 2 is dissolved in 20 ml dimethylformamide and 1ml. 10% aqueous H2SO4 is added at 40°C. HOOC-CH(OCH₂F)-COOH is isolated by crystallization under cooling and treated with SF4, in accordance with the description of W02011018466. Sevoflurane is obtained.

## Claims

1. A process for the manufacture of a substituted malonic acid derivative of formula (I) or (II):
R₁OOC-CH(OCH₂F)-COOR₂ (I)
R₃HNOC-CH (OCH₂F)-CONHR₄ (II)
wherein
- R₁, R₂ equal to or different from each other, are independently selected from H, an alkyl group having from 1 to 10 carbon atoms which is optionally substituted by at least one halogen atom, an aralkyl group or an aryl group;
- R₃, R₄ equal to or different from each other, are independently selected from H, an alkyl group having from 1 to 10 carbon atoms which is optionally substituted by at least one halogen atom, an aralkyl group or an aryl group;
by reaction of a malonic acid derivative of formula (III) or (IV):
R₁OOC-CHOH-COOR₂ (III)
R₃HNOC-CHOH-CONHR₄ (IV)
wherein
- R₁, R₂ R₃ and R₄ are as defined above,
with at least one CH₂-electrophile selected from the group consisting of (a) bis(fluoromethyl)ether and (b) formaldehyde activated with HF and at least one additional activator.

2. The process according to claim 1, wherein the CH₂-electrophile is bis(fluoromethyl)ether and the reaction is carried out in the presence of at least one additional activator.

3. The process according to claims 1 or 2, wherein the additional activator is a strong acid other than HF.

4. The process according to claim 3, wherein the additional activator is sulphuric acid.

5. The process according to anyone of claims 1 to 4, wherein the molar ratio between additional activator and formaldehyde or bis(fluoromethyl)ether is from 0,001 to 0,5.

6. The process according to anyone of claims 1 or 3 to 5, wherein the CH₂-electrophile is formaldehyde activated with HF and at least one additional activator and wherein the molar ratio HF/formaldehyde is from 1 to 10, preferably from 2 to 5.

7. The process according to anyone of claims 1 to 6 wherein the reaction is carried out in a non-protic organic solvent.

8. The process according to claim 7 wherein the solvent is selected from an amide type solvent, in particular dimethyl formamide or dimethyl acetamide, a nitrile, in particular acetonitrile, a sulphur containing solvent, in particular dimethylsulfoxide or sulfolane, a halogenated hydrocarbon such as methylene chloride, chloroform or ethylene dichloride, an ether such as diethyl ether, dibutyl ether, dioxane or tetrahydrofuran and fluorinated solvents, for example a fluorinated hydrocarbon such as in particular 1,1,1,3,3-pentafluorobutane, or a fluorinated ether such as Sevoflurane.

9. The process according to anyone of claims 1 to 6, wherein the reaction is carried out in the absence of solvent.

10. The process according to anyone of claims 1 to 9, wherein R₁ and R₂ or R₃ and R₄ are methyl or ethyl, preferably ethyl.

11. The process according to anyone of claims 1 to 10, wherein the reaction is carried out at a temperature from 0°C to 150°C, preferably from 20°C to 50°C.

12. The process according to anyone of claims 1 to 10, wherein the molar ratio between formaldehyde or bis(fluoromethyl)ether and malonic acid derivative of formula (III) or (IV) is from 1,0 to 5,0, preferably from 1.1 to 2.0.

13. The process according to anyone of claims 1 to 10, wherein the substituted malonic acid derivative of formula (I) or (II) is isolated by distillation.

14. A process for the manufacture of Sevoflurane (CF₃-CH(OCH₂F)-CF₃) which comprises (a) the manufacture of a substituted malonic acid derivative of formula (I) or (II) according to the process according to anyone of claims 1 to 13 and (b) the fluorination of the malonic acid derivative of formula (I) or (II) obtained in step (a).

15. Process according to claim 14, wherein the substituted malonic acid derivative is of formula (I) and R1 and R2 are H.
